# EUROPEAN PATENT SPECIFICATION

(11) **EP 4 326 264 B1**
(45) Date of publication and mention of the grant of the patent: **15.01.2025**
(21) Application number: 22728631.7
(22) Date of filing: 23.05.2022
(51) Int. Cl.: A61K 31/433, A61P 21/00

(54) **THIADIAZOLIDINONES FOR THEIR USE IN THE TREATMENT OF LIMB-GIRDLE MUSCULAR DYSTROPHY**
THIADIAZOLIDINONE ZUR VERWENDUNG IN DER BEHANDLUNG VON GLIEDERGÜRTEL-MUSKELDYSTROPHIE
THIADIAZOLIDINONES POUR LEUR UTILISATION DANS LE TRAITEMENT DE LA DYSTROPHIE SCAPULO-HUMÉRALE

(30) Priority: 24.05.2021 EP 21382472
(43) Date of publication of application: 28.02.2024
(73) Proprietor: Consejo Superior De Investigaciones Científicas, 28006 Madrid (ES); Administración General de la Comunidad Autónoma de Euskadi, 01010 Vitoria-Gasteiz (ES)
(72) Inventor: MARTÍNEZ GIL, Ana, 28006 Madrid (ES); PALOMO RUIZ, Valle, 28006 Madrid (ES); SÁENZ PEÑA, Miren Ametsa, 20014 San Sebastian, Guipúzcoa (ES); LÓPEZ DE MUNAIN ARREGI, Adolfo José, 20014 San Sebastian, Guipúzcoa (ES)
(74) Representative: ABG Intellectual Property Law, S.L.
(86) International application number: PCT/EP2022/063872
(87) International publication number: WO 2022/248395

(56) References cited:
- WANG MEI ET AL: "ABSTRACT", vol. 39, no. 21, 1 November 2019 (2019-11-01), US, XP055853891, ISSN: 0270-7306, Retrieved from the Internet <URL:https://journals.asm.org/doi/pdf/10.1128/MCB.00155-19> DOI: 10.1128/MCB.00155-19

## Description

### FIELD OF THE INVENTION

The present invention belongs to the field of medicinal chemistry. In particular, the invention relates to a particular type of muscular dystrophy, namely Limb-Girdle Muscular Dystrophy (LGMD), including limb girdle muscular dystrophy R1 calpain 3-related, also known as LGMDR1.

### BACKGROUND

Muscular dystrophy (MD) refers to a group of muscle diseases characterised by progressive skeletal muscle weakness, defects in muscle proteins, and the death of muscle cells and tissue. Different diseases including Duchenne, Becker, limb girdle, congenital, facioscapulohumeral, myotonic, oculopharyngeal, distal, and Emery-Dreifuss are always classified as MD but there are more than one hundred diseases in total with similarities to MD.

Most types of MD are multi-system disorders with manifestations in body systems including the heart, gastrointestinal and nervous systems, endocrine glands, skin, eyes and even brain. The condition may also lead to mood swings and learning difficulties. The symptoms usually begin during the first two decades of life and the disease gradually worsens, often resulting in loss of walking ability 10 or 20 years after onset. Subsequently, muscle degeneration progresses becoming a highly disabling disease that prevents patients from performing simple daily tasks. In Datta et al. (Current Neurology and Neuroscience Reports, 2020, 20:14) an overview on muscular dystrophies is described. Historically, these disorders are considered to be difficult to treat. However, in the last three decades, many efforts have been made in the development of novel treatments and biomarkers in the realm of muscular dystrophies commonly encountered in pediatric population.

Among the muscular dystrophy diseases, Limb-Girdle Muscular Dystrophy (LGMD) was first proposed by Walton and Nattrass in 1954 as part of a classification of muscular dystrophies. LGMD is characterised by progressive symmetrical atrophy and weakness of the proximal limb muscles and by elevated serum creatine kinase. Muscle biopsies demonstrate dystrophic lesions and electromyograms show myopathic features.

Different LGDM therapeutic strategies have been proposed *(*Current Neurology and Neuroscience Reports, 2020, 20:14), such as the use of corticosteroids (prednisone, deflazacort), anti-oxidants (epicatechin cacao flavonoid and epigallocatechin gallate green tea polyohenol), anti-fibrotic agents (CoQ.10 and lisinopril), anti-myostatin, gene-based therapies and antisense oligonucleotides. However, there is no standard of care medical therapy for LGMDs and no specific biomarkers for diagnosis other than genetic testing.

Several types of LGMD exist, including Limb-Girdle Muscular Dystrophy R1 calpain 3-related, also known as LGMDR1, LGMD2A or calpainopathy. This type is an autosomal recessive muscular dystrophy due to mutations in the CAPN3 gene that causes progressive degeneration of the proximal muscles of the pelvic and shoulder girdle.

The correct homeostasis between the synthesis and degradation of proteins in the muscle fibre is key to maintain the muscle and thus to avoid muscle atrophy and weakness (Scicchitano et al., 2018). For that purpose, there are certain signalling pathways, such as the Akt/mTOR pathway, which stimulates protein synthesis, myofibre growth and inhibits protein degradation (Glass, 2005). The ERK1/2 MAPK pathway, which controls the growth and differentiation of muscle cells, is also involved in myofibre maintenance in adults (Jones et al., 2001; Roux et al., 2004; Seaberg et al, 2015). Finally, among other pathways, the Wnt signalling participates in differentiation during muscle development and in the regeneration of muscle fibre in adults (von Maltzahn et al., 2012).

According to Jaka et al. (Expert Reviews in Molecular Medicine, 2017, 19, 1-16), two methods are used for activating the Wnt signalling pathway: genetic silencing of FRZB and administration of LiCl. Both approaches have produced an increase in β1D integrin levels that are downregulated in patients' myotubes.

The regulation of FRBZ expression is proposed as a potential therapeutic target given that *in vitro* studies support the idea that it may be possible to bring expression and phosphorylation of various proteins back towards appropriate levels in LGM DR1patients. Specifically, silencing this gene has caused the β1D integrin to return to normal levels in myotubes from LGMDR1 patients. Furthermore, since it was known that FRBZ regulates the localization of β-catenin down-stream of the Wnt pathway, it is proposed that FRZB may play a role in the crosstalk between integrin and Wnt-signalling pathway.

On the other hand, the treatment with LiCI, although with some differences, produced similar results to those obtained by siFRBZ, observing lower levels of expression of the FRBZ gene, as well as higher levels of the β1D adult isoform of the integrin in primary myotubes. Due to its activator role in the Wnt signalling pathway, certain studies showed beneficial *in vitro* results (Du et al 2009, Yang et al 2011, Abu-Baker et al 2013, Jaka et al 2017). However, the participation of lithium has only been shown in the Wnt signalling pathway, but not in other signalling pathways implicated in the development of LGMDR1. Furthermore, it is mentioned that the treatment with LiCI is associated with an inhibitory effect on GSK3β.

In fact, when the Wnt signaling pathway is active, the Wnt ligands induce the inactivation of GSK3β preventing β-catenin phosphorylation, allowing its accumulation in the cytoplasm and translocating it to the nucleus acting as a transcription factor. On the contrary, when the Wnt signaling pathway is inactive, GSK3β is activated. It phosphorylates β-catenin so that it is subsequently degraded (MacDonald et al., 2009).

Kurgan et al. (Cells, 2019, 8, 134) disclose that a low therapeutic dose of lithium (0.5 mM) has shown to inhibit GSK3 and to augment myoblast fusion, suggesting that non-toxic dose of lithium might be an effective option for promoting muscle development *in vivo.* However, no particular diseases are mentioned in this document, but some indications regarding the use of lithium for potentially attenuating some of the muscle atrophy observed in different conditions.

Whitley et al. (Physiological Reports, 2020, 8(14): e 14517) also report that low dose of LiCI treatment is effective in inhibiting GSK3β and enhances fatigue resistance in some muscles via NFAT activation.

On the other hand, some thiadiazolidinones (TDZDs) as inhibitors of GSK3β, such as tideglusib, have been synthesized (Martinez et al 2002). Tideglusib is an irreversible drug designed for the treatment of Alzheimer's disease and whose safety for treatment in humans has been demonstrated. Wang (Molecular and Cellular Biology, 2019, 39(21), e00155-19) describes the use of tideglusib for treating myotonic dystrophy, more particularly type 1 (DM1). In this case, the underlying mechanism by which tideglusib reduces DM1 pathology is the reduction of the mutant mRNA, which in turn also correct GSK3β. However, no mention is given about the particular use of tideglusib for treating LGMDR1, nor for other muscular dystrophies other than Myotonic Dystrophy.

In view of all above, no effective therapies exist for the treatment of limb girdle muscular dystrophy, and more particularly limb girdle muscular dystrophy R1 calpain 3-related (LGMDR1). In fact, the pathophysiological mechanism by which the absence of calpain 3 provokes the atrophy in muscles is not clear. Thus, there is a great need to know the alterations in the expressions of proteins involved in signalling pathways, such as Wnt and mTOR, of LGMDR1patient's muscles, and to look for new therapeutic agents to treat this disease.

### BRIEF DESRIPTION OF THE INVENTION

The authors of the present invention have found that some thiadiazolidinones (TDZDs), such as tideglusib, restore the expression and phosphorylation of key proteins involved in signalling pathways that regulate muscle homeostasis, both Wnt and mTOR pathways, and which are significantly reduced in LGMDR1 patients' muscles, thus opening the potential use of said compounds as a therapeutic option in the treatment of said disease.

Namely, the results obtained by the inventors have allowed to establish that there is a reduction of active β-catenin in the muscle of said patients confirming that Wnt pathway is altered. In addition to that, alteration of the mTOR signalling pathway has also been observed in muscle from LGMDR1 patients due to the reduced amount of the mTOR protein and its phosphorylation.

By administering the compounds in myotubes of LGMDR1patients, a complete inhibition of GSK3β has been observed which led to an increase in the amount of β-catenin and to an activation of the mTOR pathway. The restoration of said pathways supports the potential benefit of said thiadiazolidinones to be used as a therapeutic agents in the treatment for LGMDR1 patients. Furthermore, some experiments have pointed out that the treatment with said compounds is not affecting other tissues avoiding undesired adverse effects.

Thus, the main aspect of the present invention refers to 2,4-disubstituted thiadiazolidinones of formula (I): 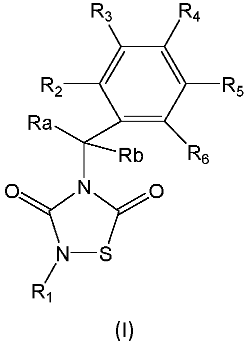 wherein:
R₁ is a substituted or unsubstituted aryl group or an alkyl group substituted with at least one aryl group;
Rₐ, R_{b}, R₂, R₃, R₄, R₅, R₆ are independently selected from hydrogen, substituted or unsubstituted alkyl, substituted or unsubstituted cycloalkyl, substituted or unsubstituted alkenyl, substituted or unsubstituted aryl, substituted or unsubstituted heterocyclyl, substituted or unsubstituted heteroaryl, -COR₇, -C(O)OR₇, -C(O)NR₇R₈ -C=NR₇, -CN, -OR₇, - OC(O)R₇, -S(O)ₜ-R₇, -NR₇R₈, -NR₇C(O)R₈, -NO₂, -N=CR₇R₈ or halogen,
t is 0, 1, 2 or 3,
R₇ and R₈ are each independently selected from hydrogen, substituted or unsubstituted alkyl, substituted or unsubstituted cycloalkyl, substituted or unsubstituted alkenyl, substituted or unsubstituted aryl, substituted or unsubstituted heterocyclyl, substituted or unsubstituted heteroaryl, substituted or unsubstituted alkoxy, substituted or unsubstituted aryloxy, halogen;
wherein Rₐ and R_{b} together can form a group =O, and wherein any pair Rₐ R₂, R₂ R₃, R₃ R₄, R₄ R₅, R₅ R₆, R₆ R_{b}, or R₇R₈ can form together a cyclic substituent;
or a pharmaceutically acceptable salt or solvate thereof,
for its use in the treatment of limb girdle muscular dystrophy.

### BRIEF DESCRIPTION OF THE FIGURES

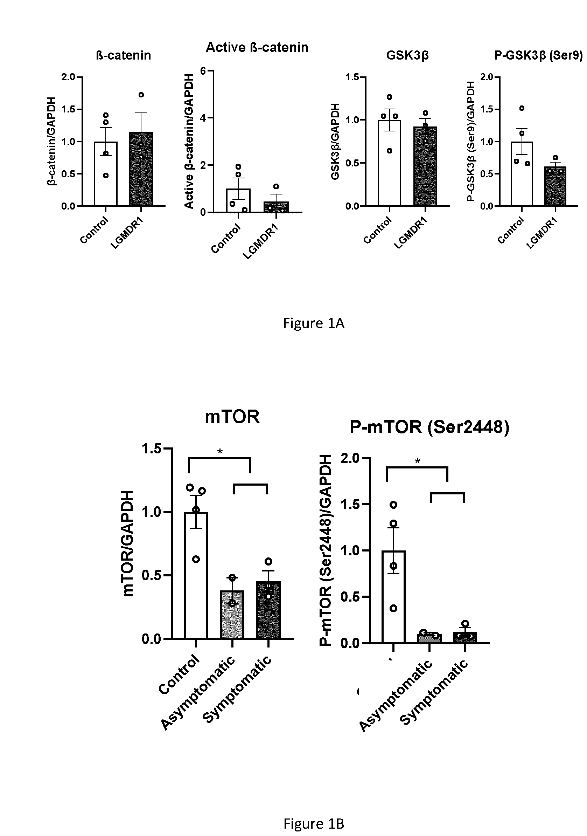

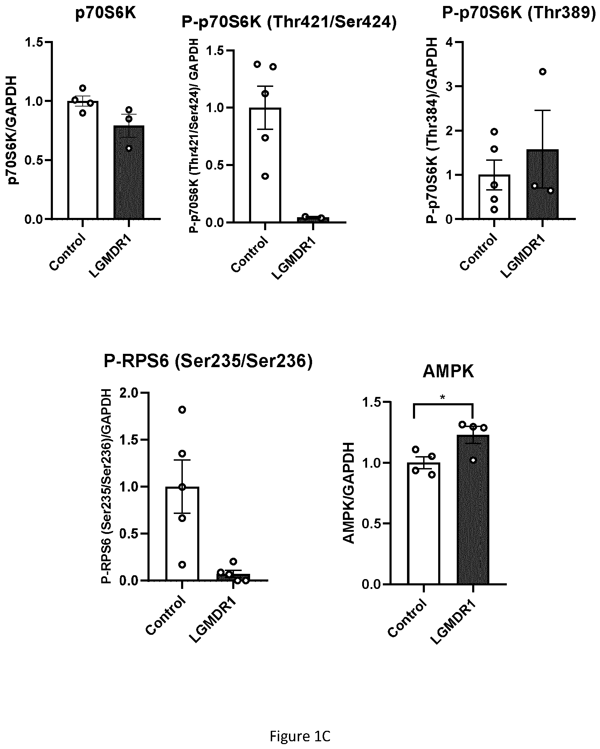

Figure 1. Quantification of Wnt and Akt/mTOR pathway proteins by Western blot in muscle from controls and LGMDR1 patients. A) Quantification of Wnt pathway proteins: β-catenin, active- β-catenin, GSK3β and P-GSK3β (Ser9); B) Quantification of Akt/mTOR pathway proteins: mTOR and P-mTOR (Ser2448). C) Detection of p70S6K, P-p70S6K (Thr421/Ser424), P-p70S6K (Thr389), P-RPS6 (Ser235/Ser236) and AMPK. Loading control: GAPDH.

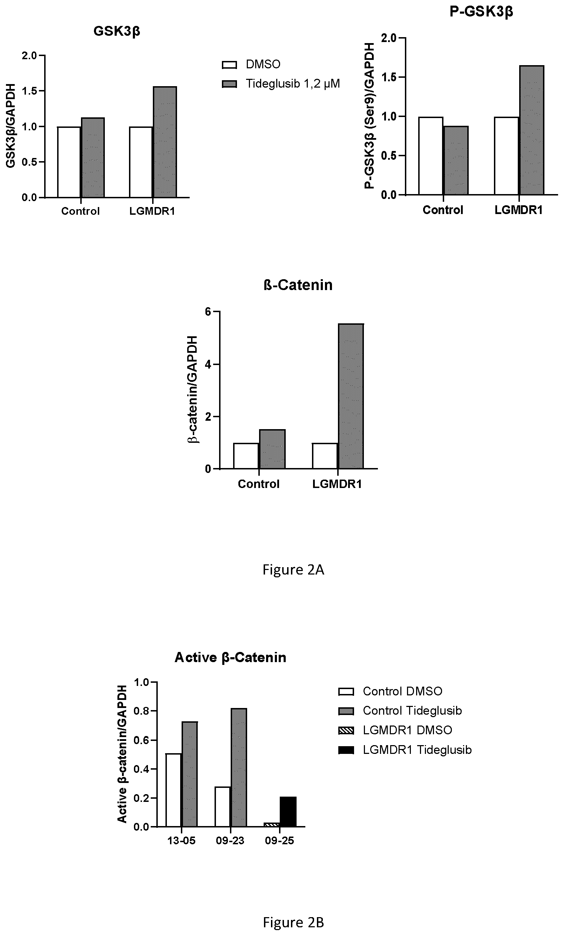

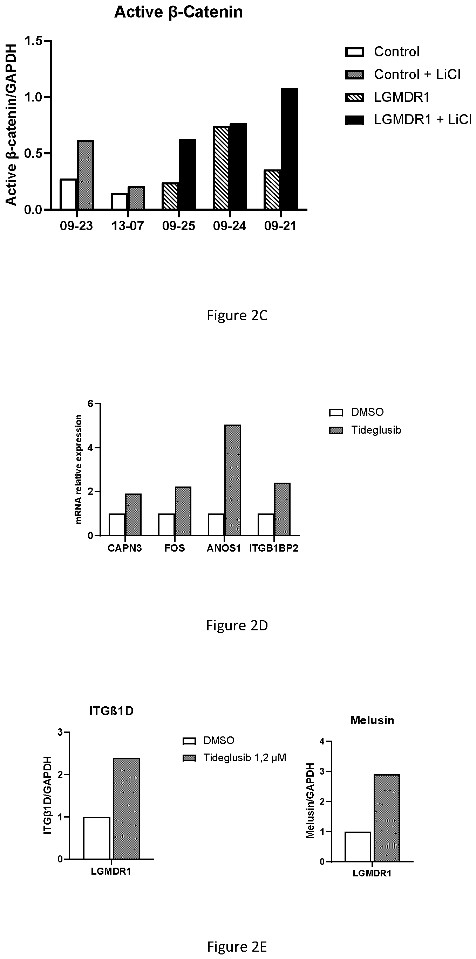

Figure 2. Activation of the Wnt pathway in human myotubes at day 8 of differentiation and after 48h of treatment with tideglusib at a concentration of 1.2 µM in control and LGMDR1 patients. A) Western blot of GSK3β, P-GSK3β (Ser9) and total β-catenin. B) Western blot of active β-catenin; C) Western blot of active β-catenin after administration of LiCI; D) Expression of CAPN3, FOS, ANOS1 and ITGB1BP2 genes in LGMDR1 myotubes. E) Western blot of structural proteins integrin β1D and melusin.

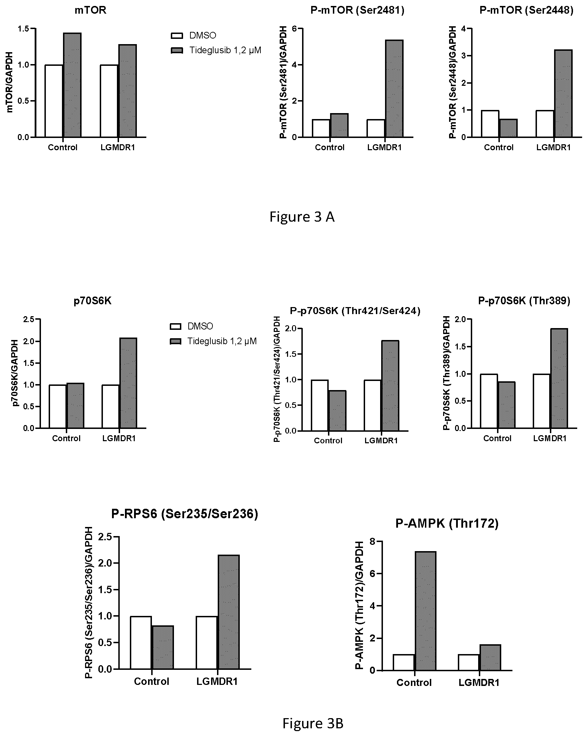

Figure 3. Activation of the Akt/mTOR pathway in human myotubes at day 8 of differentiation and after 48h of treatment with tideglusib at a concentration of 1.2 µM in control and LGMDR1 patients. A) Western blot of mTOR and P-mTOR (Ser2448 and Ser2481); B) Western blot of p70S6K, P-p70S6K (Thr421/Ser424), P-p70S6K (Thr389),P-RPS6 (Ser235/Ser236) and P-AMPK (Thr 172).

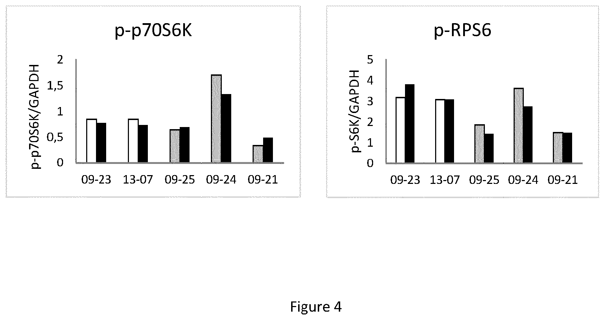

Figure 4. Analysis of the Akt/mTOR pathway in human myotubes after treatment with LiCI at a concentration of 10 mM in control and LGMDR1 patients. Densitometry results of the Western blot of the phosphorylated forms of p70S6K and RPS6 proteins. White: control samples without treatment. Grey: LGMDR1 samples without treatment. Black: LiCI treatment.

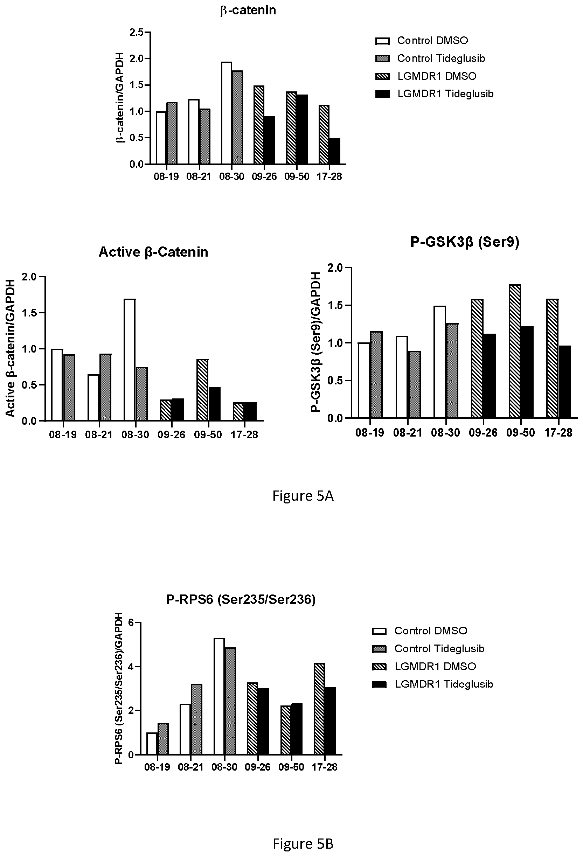

Figure 5. Analysis of the effect in protein expression on fibroblasts upon treatment with tideglusib. A) Western blot of β-catenin and its activated form. B) Western blot of p-GSK3β and P-RPS6.

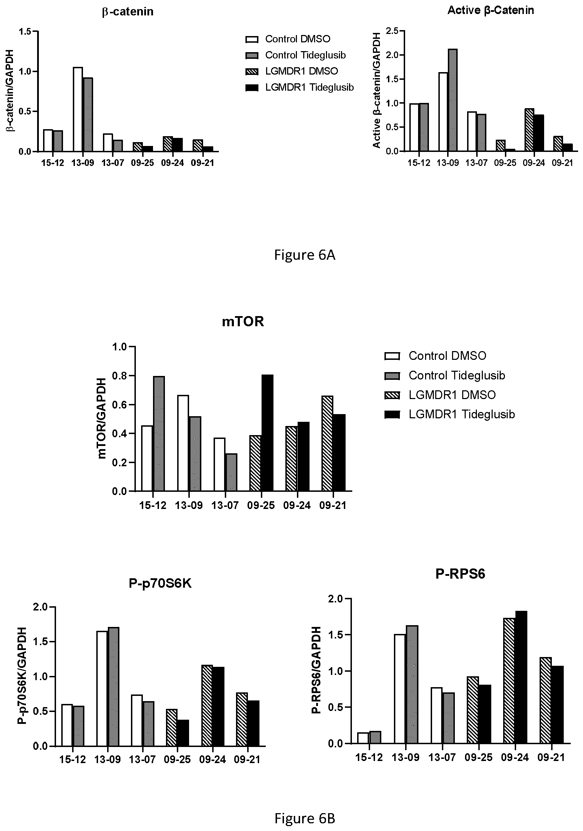

Figure 6. Analysis of the effect in protein expression on CD56-cells upon treatment with tideglusib. A) Western blot of β-catenin and its activated form. B) Western blot of mTOR, p70S6K and P-RPS6.

### DETAILED DESCRIPTION OF THE INVENTION

In the above definition of compounds of formula (I), the following terms have the meaning indicated:
"Alkyl" refers to a straight or branched hydrocarbon chain radical consisting of carbon and hydrogen atoms, containing no unsaturation, having one to six carbon atoms, more preferably one to three carbon atoms (C₁-C₃ alkyl), even more preferably one to two carbon atoms (C₁-C₂ alkyl), and which is attached to the rest of the molecule by a single bond, e. g., methyl, ethyl, n-propyl, i-propyl, n-butyl, t-butyl, n-pentyl, etc. Alkyl radicals may be optionally substituted by one or more substituents such as halogen, hydroxy, alkoxy, carboxy, cyano, carbonyl, acyl, alkoxycarbonyl, amino, nitro, mercapto and alkylthio.

"Alkenyl" refers to linear or branched hydrocarbon chain radical consisting of carbon and hydrogen atoms having at least one double bond, and from 2 to 6 carbon atoms, more preferably having 2, 3 or 4 carbon atoms. In a preferred embodiment, they refer to linear hydrocarbons having a single double bond, and from 2 to 6 carbon atoms, preferably 2 or 3 carbon atoms. Alkenyl radicals may be optionally substituted by one or more substituents such as halogen, hydroxy, alkoxy, carboxy, cyano, carbonyl, acyl, alkoxycarbonyl, amino, nitro, mercapto and alkylthio.

"Aryl" refers to an aromatic group having between 6 to 18 carbon atoms, preferably between 6 and 12 carbon atoms, more preferably between 6 and 10 and even more preferable having 6 carbon atoms, comprising 1, 2 or 3 aromatic nuclei, including for example and in a non-limiting sense, phenyl, biphenyl, naphthyl, indenyl, phenanthryl, anthracyl or terphenyl. Preferably, aryl refers to phenyl (Ph) or naphthyl. The aryl radical may be optionally substituted by one or more substituents such as hydroxy, mercapto, halogen, alkyl, phenyl, alkoxy, haloalkyl, aralkyl, nitro, cyano, dialkylamino, aminoalkyl, acyl and alkoxycarbonyl, as defined herein. The aryl group can also be fused to another non-aromatic ring, such as a cycloalkyl or a heterocyclyl.

"Cycloalkyl" refers to a saturated or partially saturated mono- or bicyclic aliphatic group having between 3 and 10, preferably between 3 and 6 carbon atoms ("C₃-C₆ cycloalkyl"), which is bound to the rest of the molecule by means of a single bond, including for example and in a non-limiting sense, cyclopropyl, cyclohexyl or cyclopentyl. Unless otherwise stated specifically in the specification, the term "cycloalkyl" is meant to include cycloalkyl radicals which are optionally substituted by one or more such as alkyl, halo, hydroxy, amino, cyano, nitro, alkoxy, carboxy and alkoxycarbonyl.

"Heterocyclyl" refers to a stable 3-to 15 membered monocyclic or bicyclic system which can be partially or fully saturated containing 5 to 15, preferably 5 or 6, ring atoms containing one or more, specifically one, two, three, or four ring heteroatoms independently selected from N, O, and S, preferably one or two, and the remaining ring atoms being carbon. Examples of such heterocycles include, but are not limited to, pyrrolidine, piperidine, tetrahydropyridine, piperazine, morpholine, thiomorpholine, azepane, diazepane, tetrahydrofuran, tetrahydropyran, octahydro-pyrrolopyrazine.

"Heteroaryl" refers to a monocyclic or bicyclic system which is aromatic and contains 5 to 18, preferably 5 or 6, ring atoms containing one or more, specifically one, two, three, or four ring heteroatoms independently selected from N, O, and S, preferably one or two, and the remaining ring atoms being carbon. Examples of such heteroaryl include, but are not limited to, thiophene, furan, pyrrole, thiazole, oxazole, isothiazole, isooxazole, imidazole, pyrazole, triazole, oxadiazole, thiadiazole, tetrazole, tetrazole oxide, oxadiazolone, pyridine, pyrimidine, pyrazine, dihydroindolone, benzimidazole, isoindole, benzothiazole, benzofuran, indole, purine, quinolone, isoquinoline.

"Aralkyl" refers to an aryl group as defined above linked to an alkyl group as defined above. Preferred examples include benzyl and phenethyl.

"Halogen" refers to bromo, chloro, iodo or fluoro.

"Carboxy" refers to a radical of the formula -C(O)OH.

"Alkoxy" refers to a radical of the formula -ORₐ where Rₐ is an alkyl radical as defined above, e. g., methoxy, ethoxy, propoxy, etc.

"Alkoxycarbonyl" refers to a radical of the formula -C(O)ORₐ where Rₐ is an alkyl radical as defined above, e. g., methoxycarbonyl, ethoxycarbonyl, propoxycarbonyl, etc.

"Amino" refers to a radical of the formula-NH₂, -NHRₐ or -NRₐR_{b}, wherein Rₐ and R_{b} are alkyl radicals as defined above.

"Alkylthio" refers to a radical of the formula -SRₐ where Rₐ is an alkyl radical as defined above, e. g., methylthio, ethylthio, propylthio, etc.

"Acyl" refers to a radical of the formula-C(O)-Rc and -C(O)-Rd, where Rc is an alkyl radical as defined above and Rd is an aryl radical as defined above, e. g., acetyl, propionyl, benzoyl, and the like.

"Haloalkyl" refers to an alkyl radical, as defined above, that is substituted by one or more halogen radicals, as defined above, e. g., trifluoromethyl, trichloromethyl, 2,2,2-trifluoroethyl,1-fluoromethyl-2-fluoroethyl, and the like.

The term "pharmaceutically acceptable salts or solvates" refers to any pharmaceutically acceptable salt or solvate which, upon administration to the recipient, is capable of providing (directly or indirectly) a compound as described herein. The preparation of salts and solvates can be carried out by methods known in the art.

For instance, pharmaceutically acceptable salts of compounds used in the present invention are synthesized from the parent compound which contains a basic or acidic moiety by conventional chemical methods. Generally, such salts are, for example, prepared by reacting the free acid or base forms of these compounds with a stoichiometric amount of the appropriate base or acid in water or in an organic solvent or in a mixture of the two.

The compounds used in the present invention may be in crystalline form either as free compounds or as solvates (e.g. hydrates). Methods of solvation are generally known within the art. In a particular embodiment the solvate is a hydrate.

The compounds of formula (I), or their salts or solvates, are preferably used in pharmaceutically acceptable or substantially pure form. By pharmaceutically acceptable form is meant, inter alia, having a pharmaceutically acceptable level of purity excluding normal pharmaceutical additives such as diluents and carriers, and including no material considered toxic at normal dosage levels. Purity levels for the drug substance are preferably above 50%, more preferably above 70%, most preferably above 90%. In a preferred embodiment it is above 95% of the compound of formula (I), or of its salts or solvates.

The compounds used in the present invention represented by the above described formula (I) may include enantiomers depending on the presence of chiral centres or isomers depending on the presence of multiple bonds (e.g. Z, E). The single isomers, enantiomers or diastereoisomers and mixtures thereof can be used in the present invention.

In a preferred embodiment, R₁ is selected from:
- a C₆-C₁₀ aryl group, optionally substituted with at least one C₁-C₆ alkyl group, an aryloxy group, or an aralkyl group, or fused to a non-aromatic ring; and
- a C₁-C₃ alkyl group substituted with at least one C₆-C₁₀ aryl group, wherein the aryl group is optionally substituted with at least one C₁-C₃ alkyl group or C₁-C₃ alkoxy group.

Also preferred are compounds in which R₁ is a C₆-C₁₀ aryl group directly linked to the N of the thiadiazolidine, wherein said C₆-C₁₀ aryl group is optionally substituted with at least one C₁-C₆ alkyl group, an aryloxy group, or an aralkyl group, or fused to a non-aromatic ring.

Most preferred are those compounds wherein R₁ is a non-substituted C₆-C₁₀ aryl group.

Representative substituents that can be used as R₁ are the following:

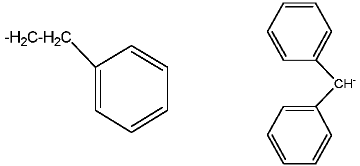

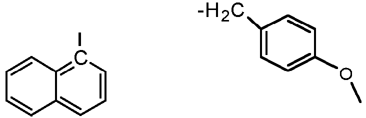

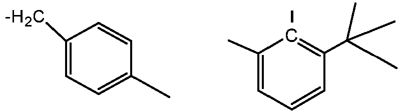

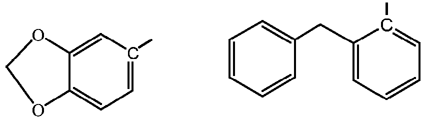

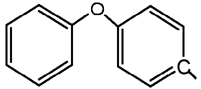

In a particular embodiment, compounds in which R₁ is a naphthyl group are preferred, most preferably if R₁ is a α-naphthyl group.

In another preferred embodiment, Rₐ and R_{b} are H.

In another preferred embodiment, R₂, R₃, R₄, R₅, R₆ are independently selected from hydrogen, substituted or unsubstituted alkyl, COR₇, -C(O)OR₇, -OR₇, -NR₇R₈, or halogen, wherein R₇ and R₈ are each independently selected from hydrogen, substituted or unsubstituted alkyl, substituted or unsubstituted cycloalkyl, substituted or unsubstituted alkenyl, substituted or unsubstituted aryl, substituted or unsubstituted heterocyclyl, substituted or unsubstituted heteroaryl, substituted or unsubstituted alkoxy, substituted or unsubstituted aryloxy, halogen.

More preferably, R₂, R₃, R₄, R₅ and R₆ are H.

Even most preferably, Rₐ, R_{b}, R₂, R₃, R₄, R₅ and R₆ are H.

Representative compounds used in the present invention are the following: 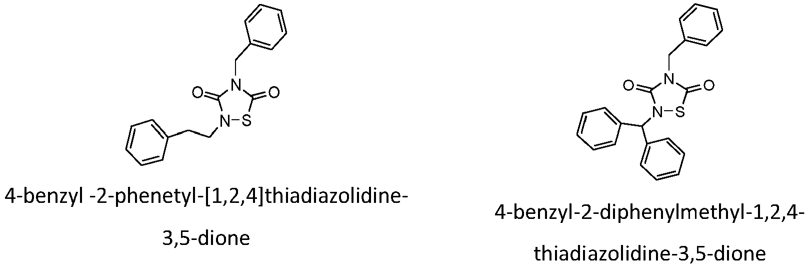 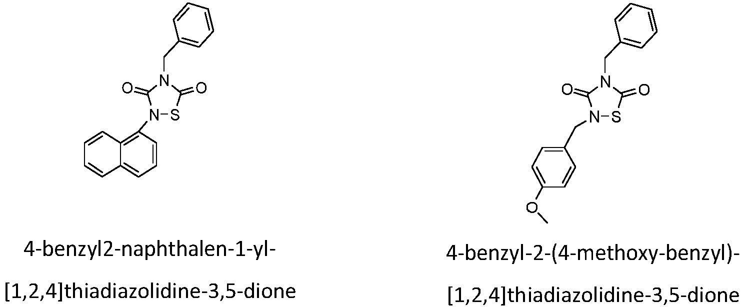 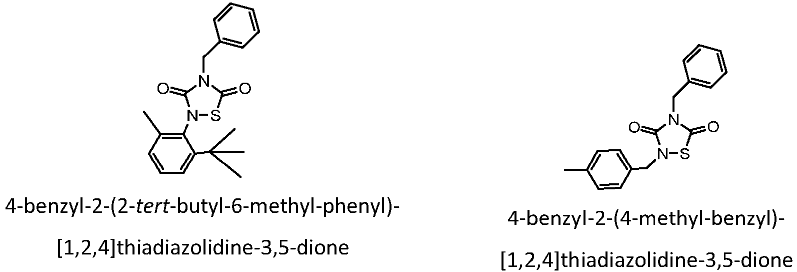 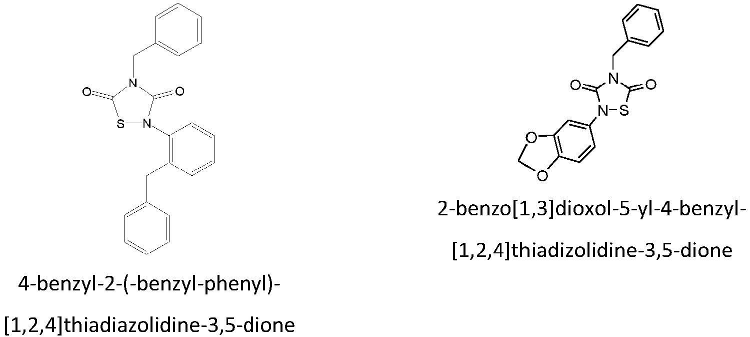 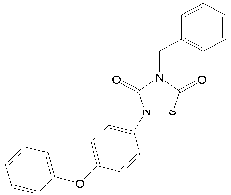 4-benzyl-2-(4-phenoxy-phenyl)-[1,2,4]thiadiazolidine-3,5-dione
and their salts or solvates.

The most preferred compound is: 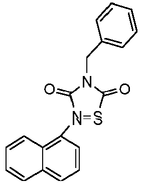 4-benzyl2-naphthalen-1-yl-[1,2,4]thiadiazolidine-3,5-dione, also known as tideglusib.

The compounds of formula (I) defined above and used in the present invention can be obtained by available synthetic procedures. Some examples of these procedures are described in WO 01/85685, US 2003/0195238 and WO2005/097117 and references therein.

In a particular embodiment, the limb girdle muscular dystrophy includes both recessive limb girdle muscular dystrophies (LGMD R) and dominant limb girdle muscular dystrophies (LGMD D).

Within this particular embodiment, recessive limb girdle muscular dystrophies are selected from LGMD R1 calpain3-related (LGMDR1 or calpainopathy), LGMD R2 dysferlin-related (LGMDR2), LGMD R3 α-sarcoglycan-related (LGMDR3), LGMD R4 β-sarcoglycan-related (LGMDR4), LGMD R5 γ-sarcoglycan-related (LGMDR5), LGMD R6 δ-sarcoglycan-related (LGMDR6), LGMD R7 telethonin-related (LGMDR7), LGMD R8 TRIM 32-related (LGMDR8), LGMD R9 FKRP-related (LGMDR9), LGMD R10 titin-related (LGMDR10), LGMD R11 POMT1-related (LGMDR11), LGMD R12 anoctamin5-related (LGMDR12), LGMD R13 Fukutin-related (LGMDR13), LGMD R14 POMT2-related (LGMDR14), LGMD R15 POMGnT1-related (LGMDR15), LGMD R16 α-dystroglycan-related (LGMDR16), LGMD R17 plectin-related (LGMDR17), LGMD R18 TRAPPC11-related (LGMDR18), LGMD R19 GMPPB-related (LGMDR19), LGMD R20 ISPD-related (LGMDR20), LGMD R21 POGLUT1-related (LGMDR21), LGMD R22 collagen6-related (LGMDR22), LGMD R23 laminin α2-related (LGMDR23), LGMD R24 POMGNT2-related (LGMDR24).

Within said particular embodiment, dominant limb girdle muscular dystrophies are selected from LGMD D1 DNAJB6-related (LGMD D1), LGMD D2 TNP03-related (LGMD D2), LGMD D3 HNRNPDL-related (LGMD D3), LGMD D4 calpain3-related (LGMD D4) and LGMD D5 collagen6-related (LGMD D5).

The different types of limb girdle muscular dystrophies that have been cited are in accordance to the new nomenclature agreed at the 229^{th} ENMC International workshop *(*Neuromuscular Disorders, 2018, 28, 702-710). This classification follows the formula: LGMD, inheritance (R for recessive and D for dominant), order of discovery (number) and affected protein. In a preferred embodiment, the limb girdle muscular dystrophy, is limb-girdle muscular dystrophy R1 calpain 3-related, also referred to as LGMDR1 or calpainopathy.

The terms "treating" and "treatment", as used herein, means reversing, alleviating, inhibiting the progress of, the disease or condition to which such term applies, or one or more symptoms of such disease or condition, such as lowering the viral load in a patient with respect to pretreatment levels.

The term "subject" refers to a mammal, e.g., a human.

In particular, the compounds for use according to the invention are administered as a pharmaceutical composition, which comprises the corresponding active compound of formula (I) and one or more pharmaceutically acceptable excipients.

The term "pharmaceutically acceptable excipient" refers to a vehicle, diluent, or adjuvant that is administered with the active ingredient. Such pharmaceutical excipients can be sterile liquids, such as water and oils, including those of petroleum, animal, vegetable, or synthetic origin, such as peanut oil, soybean oil, mineral oil, sesame oil, and similars. Water or saline aqueous solutions and aqueous dextrose and glycerol solutions, particularly for injectable solutions, are preferably used as vehicles. Suitable pharmaceutical vehicles are known by the skilled person. The pharmaceutically acceptable excipient necessary to manufacture the desired pharmaceutical composition of the invention will depend, among other factors, on the elected administration route. Said pharmaceutical compositions may be manufactured according to conventional methods known by the skilled person in the art.

The compounds for use according to the invention may be administered in a "therapeutically effective amount", i.e. a nontoxic but sufficient amount of the corresponding compound to provide the desired effect. The amount that is "effective" will vary from subject to subject, depending on the age and general condition of the individual, the particular compound administered, and the like. However, an appropriate amount in any individual case may be determined by one of ordinary skill in the art using routine experimentation.

The compounds for use according to the invention may be administered by any appropriate route (via), such as oral (e.g., oral, sublingual, etc.) or parenteral (e.g., subcutaneous, intramuscular, intravenous, etc.).

Examples of pharmaceutical compositions include any solid (tablets, pills, capsules, granules etc.) or liquid (solutions, suspensions or emulsions) composition for oral or parenteral administration.

In a preferred embodiment the pharmaceutical compositions are in oral form. Suitable dose forms for oral administration may be tablets and capsules and may contain conventional excipients known in the art such as binding agents, for example syrup, acacia, gelatin, sorbitol, tragacanth, or polyvinylpyrrolidone; fillers, for example lactose, sugar, maize starch, calcium phosphate, sorbitol or glycine; tabletting lubricants, for example magnesium stearate; disintegrants, for example starch, polyvinylpyrrolidone, sodium starch glycollate or microcrystalline cellulose; or pharmaceutically acceptable wetting agents such as sodium lauryl sulfate.

The solid oral compositions may be prepared by conventional methods of blending, filling or tabletting. Repeated blending operations may be used to distribute the active agent throughout those compositions employing large quantities of fillers. Such operations are conventional in the art. The tablets may for example be prepared by wet or dry granulation and optionally coated according to methods well known in normal pharmaceutical practice, in particular with an enteric coating.

The pharmaceutical compositions may also be adapted for parenteral administration, such as sterile solutions, suspensions or lyophilized products in the appropriate unit dosage form. Adequate excipients can be used, such as bulking agents, buffering agents or surfactants.

The mentioned formulations will be prepared using standard methods such as those described or referred to in the European and US Pharmacopoeias and similar reference texts.

The compounds for use according to the invention will typically be administered once or more times a day, for example 1, 2, 3 or 4 times daily, with typical total daily doses depending on the particular compound and severity of the disease, and may be easily determined by the skilled practitioner.

By way of example, typical total daily doses the compounds of the invention are in the range of from 0.1 to 1000 mg/day, preferably from 1 to 600 mg/ day, even more preferably from 1 to 100 mg/day.

The compounds and compositions for use according to the invention may be administered as the sole active ingredient, or in combination with other active ingredients to provide a combination therapy. The other active ingredients may form part of the same composition, or be provided as a separate composition for administration at the same time or at different time.

In a particular embodiment, the compounds are used in combination with one or more compounds useful in the treatment of limb girdle muscular dystrophy, more preferably calpainopathy or limb-girdle muscular dystrophy R1 calpain 3-related (LGMDR1).

The term "combination" refers to a product comprising one or more of the defined compounds, either in a single composition or in several compositions (or units), in which case the corresponding compounds are distributed among the several compositions. Preferably, the combination refers to several compositions, in particular comprising one composition (or unit) per compound (compound as defined above) of the combination. The expression "one or more" when characterizing the combination refers to at least one, preferably 1, 2, 3, 4, or 5 compounds, more preferably, 1, 2 or 3 compounds, even more preferably 1 or 2 compounds.

When the combination is in the form of a single composition, the compounds present in the combination are always administered simultaneously.

When the combination is in the form of several compositions (or units), each of them having at least one of the compounds of the combination, the compositions or (units) may be administered simultaneously, sequentially or separately.

Simultaneous administration means that the compounds or compositions (or units) are administered at the same time.

Sequential administration means that the compounds or compositions (or units) are administered at different time points in a chronologically staggered manner.

Separate administration means that the compounds or compositions (or units) are administered at different time points independently of each other.

In particular, the combinations for use according to the invention are administered as pharmaceutical compositions, which comprise the corresponding (active) compounds and a pharmaceutically acceptable excipient, as previously defined.

The combinations for use according to the invention will typically be administered once or more times a day, for example 1, 2, 3 or 4 times daily, with typical total daily doses depending on the particular compound and severity of the disease, and may be easily determined by the skilled practitioner.

The following examples are intended to further illustrate the invention. They should not be interpreted as a limitation of the scope of the invention as defined in the claims.

### Examples

### Materials and Methods

### Muscle biopsy samples and primary cell culture

All participants gave informed consent for sample collection (Table 1 below). Proximal muscle samples (quadriceps, deltoid, biceps and triceps) were obtained from healthy controls and LGMDR1 patients, in whom the diagnosis was genetically confirmed by identification of the two mutations in the calpain 3 gene. One fraction of the biopsies was used to obtain protein, the other to obtain myoblasts.

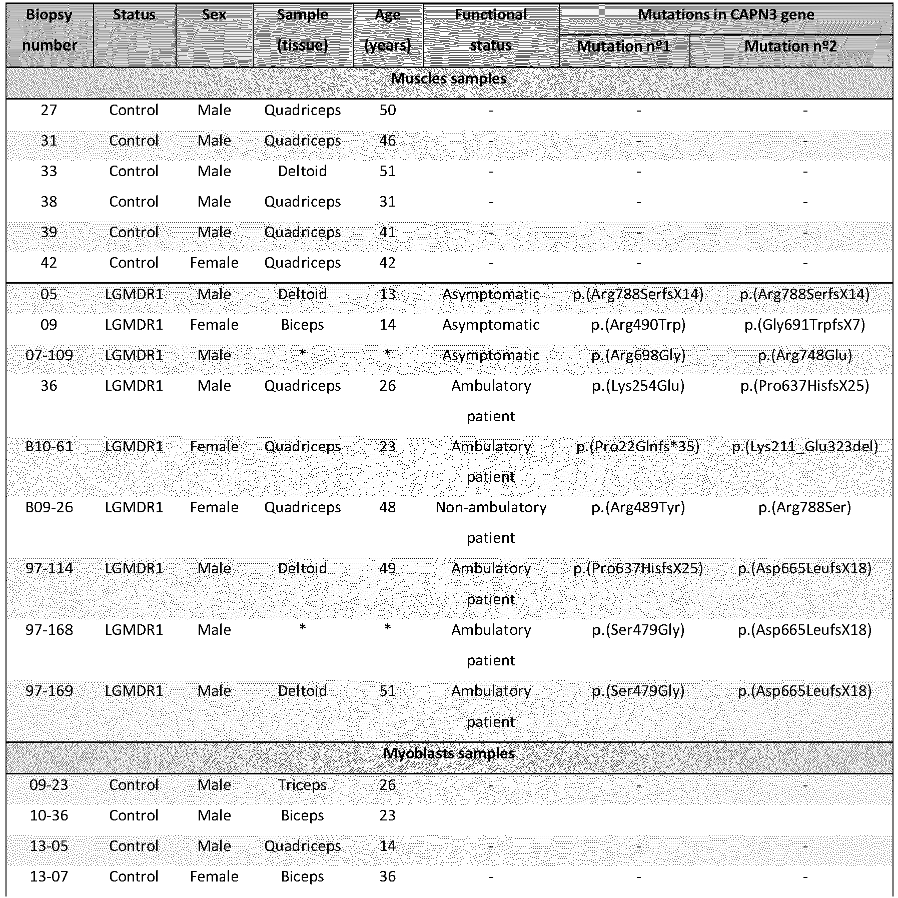

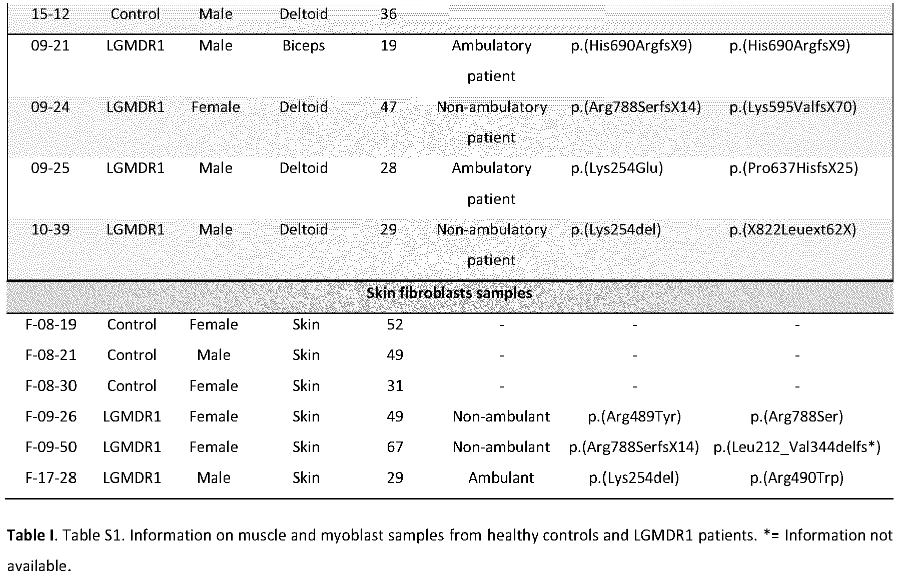

To obtain primary myoblasts, muscle biopsies were processed and cultured in monolayer according to the method described by Askanas (Neurology, 1975, 25, 58-67). To isolate the pure population of myoblasts and CD56-negative cells (CD56-), the protocol described previously (Jaka et al., Expert Reviews in Molecular Medicine, 2017, 19, e2) was used, using immunomagnetic separation based on the early presence of the surface marker CD56 (Miltenyi Biotec, 130-050-401).

Myoblasts were cultured in proliferation medium (DMEM, M-199, Insulin, Glutamine and penicillin-streptomycin) with 10% FBS and growth factors at 37 °C in 5% CO₂ air incubator. When myoblasts reached confluence, the FBS concentration was reduced to 2% and growth factors were removed from the medium, promoting fusion and production of myotubes.

### Skin biopsies and fibroblast culture

Skin fibroblasts from healthy controls and from LGMDR1 patients were provided by Dr. Ana Aiastui (Biodonostia Health Research Institute, Cell Culture and Histology Platform). Cells were cultured in Dulbecco's modified Eagle's medium (DMEM, Gibco, 41966-029) supplemented with 10% FBS (Gibco, 10270-106) and 1% penicillin-streptomycin (Gibco, 15140-122) at 37 °C in a 5% CO₂ air incubator. Human fibroblasts were cultured at a density of 4×10⁴ cells/ml.

### FRZB gene silencing

FRZB gene silencing in primary human myotubes has been performed using interfering RNA as previously described (Jaka et al., Expert Reviews in Molecular Medicine, 2017, 19, e2). The siRNA for FRZB gene silencing (s5369) and the siRNA used as negative control (AM4611) were obtained from Life Technologies. Cells plated at 24 000 cells/cm² were transfected with the siRNA at a concentration of 5 nM using RiboCellin transfection reagent (Eurobio) following the manufacturer's instructions. After 8 days of differentiation, human primary myotubes were incubated with the corresponding siRNA and the transfection agent. Finally, the RNA obtained from these cultures was analysed 48 h post-transfection by quantitative real-time PCR. Likewise, proteins from these cultures were analysed 72 h post-transfection.

### Administration of compounds

Tideglusib was provided by Dr. Ana Martinez (from The Center for Biological Research, CSIC). It was administered at 1.2 µM, whereas LiCI (Sigma-Aldrich, L7026) was used for comparative purposes at a final concentration of 10 mM. Tideglusib and LiCI were administered at the above referred concentrations to myotubes at eight days of differentiation, and tideglusib also to skin fibroblasts and to CD56- cells. Negative controls, in the case of tideglusib, were performed with DMSO (Fisher Scientific, BP-231). At 48 hours after drug administration, RNA and proteins were extracted.

### RNA extraction from myoblast/myotubes and muscle biopsies

RNA extraction from primary myoblast/myotubes was performed with an RNeasy Mini Kit (Qiagen). In the case of the muscle biopsies, which were snap frozen and stored at -80°C until use, total RNA was obtained using an RNA-Plus Kit (QBiogene).

### Quantitative real-time PCR

The isolated RNA was reverse-transcribed to first-strand complementary DNA (cDNA) in a final volume of 50 µl using a High Capacity cDNA Reverse Transcription Kit (Applied Biosystems), according to the manufacturer's instructions. The expression levels of CAPN3, FOS, ITGB1BP2, ANOS1 and GAPDH genes in the samples were assessed by real-time quantitative PCR with TaqMan probes (Table II below) using the 'CFX384 Real-Time PCR Detection System' (Bio-Rad).

**Table II. TaqMan probes.**

| **Gene and exon to which the probe binds** | **Probe's code** |
|---|---|
| *CAPN3* Ex1-2 | Hs00181057_m1 |
| FOS Ex1-2 | Hs99999140_m1 |
| *ITG818P2* Ex4-5 | Hs00183746_m1 |
| *ANOS1* Ex2-3 | Hs01085107_m1 |
| *GAPDH* Ex2 | Hs99999905_m1 |

### Protein extraction and Western blot analysis

Protein collection from muscle samples and cell cultures, as well as Western blot procedure, were performed as previously described (Jaka et al., Expert Reviews in Molecular Medicine, 2017, 19, e2). The antibodies used are listed in Table III below.

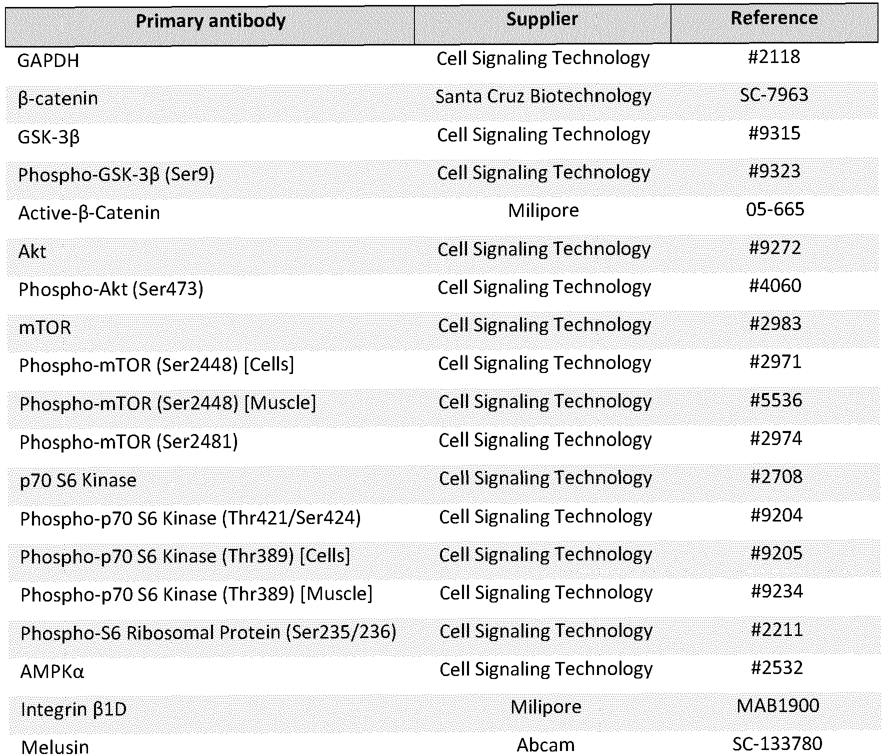

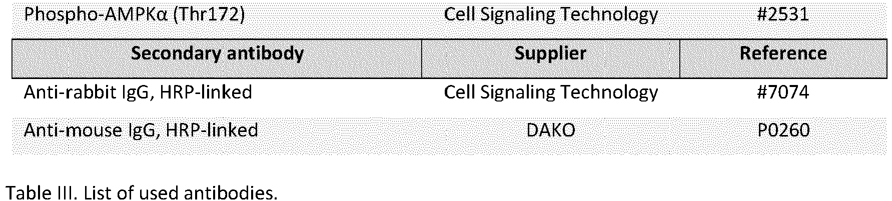

### Example 1. Alterations in Wnt and mTOR pathways involved in LGMDR1

### 1. Alteration of the Wnt/ 6-catenin pathway in the muscle of LGMDR1 patients

Previous studies had described the overexpression of FRZB, a Wnt1, 5a, 8 and 9a antagonist, in the muscle of LGMDR1 patients (Jaka et al., ExpertReviews in Molecular Medicine, 2017, 19, e2). Due to the excessive inhibition of this pathway, the expression of some proteins participating in the Wnt pathway has been analysed.

Namely, the levels of GSK3β and total β-catenin were tested, although no significant differences were observed between patients and controls. However, the active form of β-catenin, as well as the phosphorilation form of GSK3 β (Ser9), showed a drastic decrease of these proteins in patients (Figure 1A). These results have allowed establishing that in the muscle of LGMDR1 patients there is a reduction of active β-catenin, which confirms that the Wnt pathway is altered.

### 2. Reduction in the activation of the mTOR pathway in the muscle of LGMDR1 patients

The expression of mTOR as well as its phosphorylated form in Ser2448 were severely reduced in muscle of LGMDR1 patients (p=0.00159; p=0.00159 respectively). The decrease of both forms can be observed in pseudoasymptomatic and symptomatic patients (Figure 1B).

Subsequently, the expression of kinases downstream of this pathway was analysed. p70S6K is a kinase participating in the mTOR pathway and that is activated by sequential phosphorylations (Pullen et al., FEBS Letters, 1997, 410(1), 78-82; Fenton et al., The International Journal of Biochemistry & Cell Biology, 2011, 43(1), 47-59). Our results show that the levels of its phosphorylation form at the Thr421/Thr424 residues (Figure 1C) showed a greatly decreased. Downstream, the kinase p70S6K catalyzes the phosphorylation of ribosomal protein S6 (RPS6), whose phosphorylation correlates with high levels of protein translation and synthesis (Nielsen et al. The Journal of Biological Chemistry, 1982, 257(20), 12316-12321; Fenton et al., The International Journal of Biochemistry & Cell Biology, 2011, 43(1), 47-59). When analysing the phosphorylation of RPS6 (Ser235/Ser236), a statistically significant decrease was found in the muscle of LGMDR1 patients when analyzed together (p = 0.03, data not shown, and Figure 1C).

On the other hand, being known that AMPK kinase is an inhibitor of the mTOR pathway (Du et al., Biochemical and Biophysical Research Communications, 2008, 368(2), 402-407; Hwang et al., British Journal of Pharmacology, 2013, 169(1), 69-81), it was analysed in the muscle of patients and an increase in the amount of this kinase was observed in the group of patients (p = 0.038) (Figure 1C).

These results point out that alterations in the mTOR signalling pathway are also present in muscle from LGMDR1 patients, since severe decrease in mTOR expression, its phosphorylation, as well as a reduction in the activation of downstream proteins of the pathway have been observed, which leads to dysregulation of the growth of muscle fibre.

### Example 2. In vitro treatment with tideglusib

Tideglusib was administered according to the procedure mentioned above in different cell types: primary myotubes, skin fibroblasts and CD56-cells. Li has also been used for comparative purposes.

The effect on the response for the administered drugs was different depending on the cell type as shown below.

### 2.1.Activation of the Wnt pathway in human myotubes and recovery of deregulated gene expression in LGMDR1 patients

We proceeded to analyse the expression of the GSK3β protein, as well as its downstream effect on the Wnt pathway in myotubes from controls and patients after drug administration. Inhibition by tideglusib increases the amount of total GSK3β and its phosphorylation (Ser9) in patients. The activation of the Wnt pathway was confirmed by effective inhibition of GSK3β kinase, since an increase in total β-catenin expression (Figure 2A) as well as active β-catenin (Figure 2B) was observed with tideglusib. As a control assay, lithium was administered and an increase in active β-catenin was verified (Figure 2C).

On the other hand, in previous studies we had already described that activating the Wnt pathway through the silencing of the FRZB gene or with treatment with lithium, the expression of some altered genes in the muscle of patients was recovered (Jaka et al., Expert Reviews in Molecular Medicine, 2017, 19, e2; Casas -Fraile et al., Orphanet Journal of Rare Diseases, 2020, 15(1), 119). Thus, their expression was analysed after administering tideglusib. The results showed a correlation with the effect generated by the FRZB gene silencing, that is, an increase in the expression of the CAPN3, FOS and ANOS1, and an increase in ITGB1BP2 was also observed (Figure 2D).

Likewise, the expression of some of the structural proteins that are part of the costamere, whose expression is altered in LGMDR1 patients, was analysed. After drug administration, an increase in integrin β1D and melusin was also observed (Figure 2E).

### 2.2. Activation of theAkt/mTOR pathway in myotubes from LGMDR1 patients

After administration of tideglusib in the patient's myotubes, no significant changes were observed in total mTOR, but there was a significant increase in phosphorylation of mTOR in two of its residues (Ser2448 and Ser2481) (Figure 3A).

Furthermore, downstream of the mTOR pathway, the p70S6K and RPS6 proteins were analysed. After administration of tideglusib, the expression of total p70S6K, its phosphorylation (Thr421/Ser424 and Thr389), as well as the levels of phosphorylation of the RPS6 protein (Ser235/Ser236) increased in the patient's sample. In the control, the effect observed after the treatments is very slight (Figure 3B).

These results have shown that the administration of a compound of formula (I) as the ones described above, and particularly tideglusib, in myotubes increases the amount of β-catenin which in turn leads to an activation of the Wnt pathway, as well as also activates the mTOR pathway.

For comparative purposes, LiCI was also administered in control and patient's myotubes and the phosphorylation of p70S6K and RPS6 proteins were also tested. The results are depicted in Figure 4.

It can be observed that the administration of lithium did not provide any increase in the proteins involved in the mTOR pathway, nor in any of their phosphorylation forms, thus confirming that this compound is not effective for regulating one of the most important signalling pathways involved in LGMDR1 in spite of having been described its use in the treatment of this disease in the prior art.

The comparative results between lithium and tideglusib are also expressed in Tables IV and V, wherein the increase in the expression of p70S6K, its phosphorylation (Thr421/Ser424 and Thr389), and in the levels of phosphorylation of the RPS6 protein upon administration of tideglusib in patient's sample is observed when compared to the administration of lithium.

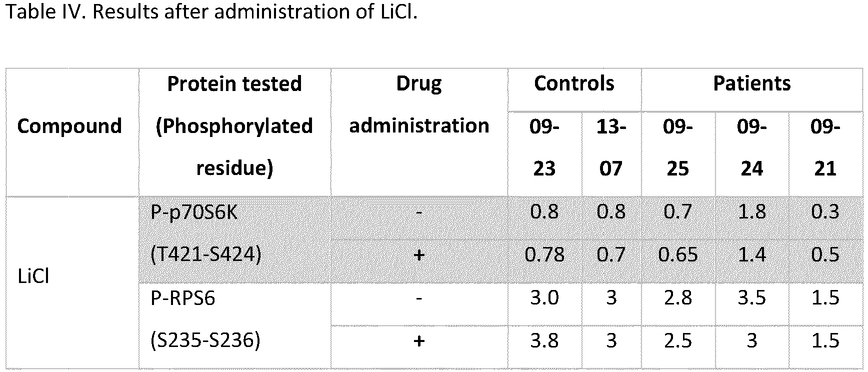

**Table V. Results after administration of tideglusib.**

| **Compound** | **Protein tested (Phosphorylated residue)** | **Control 13-05** | **Patient 09-25** |
|---|---|---|---|
| Tideglusib | P-p70S6K (T421-S424) | 0.8 | 1.8 |
| | P-p70S6K (T389) | 0.85 | 1.8 |
| | P-RPS6 (S235-S236) | 0.8 | 1.6 |

*The values shown in the table correspond to the protein expression value relative to that of the same sample without treatment.*

Thus, the fact that compounds of formula (I), such as tideglusib, recover both, the Wnt and mTOR signalling pathways in LGMDR1 patients is of great importance for the treatment of this disease as they can be more effective than other known compounds to recover the altered homeostasis of the muscle fibre.

### 2.3. Absence of activation of the Wnt pathway in fibroblasts and CD56- after treatment with tideglusib

In order to establish whether the effects previously observed after drug administration in myogenic cells (myoblasts differentiated to myotubes) occurred similarly in other cell types, we proceeded to study skin fibroblasts and CD56- cells obtained from patients' muscle.

### Fibroblasts

Treatment with tideglusib in fibroblasts did not show an increase in the phosphorylation of GSK3β (Ser9) (Figure 5A), nor in β-catenin or its active form, indicating that the Wnt pathway is not activated in this cell type by means of tideglusib. Furthermore, the effect that the inhibition of GSK3β could cause in the mTOR pathway was also tested, even observing a decrease in the phosphorylation of RPS6 (Figure 5B).

### CD56- cells

After treatment with tideglusib in CD56-cells, no significant alteration was observed in the amount of total β-catenin or in its active form (Figure 6A). Finally, the effect of this inhibition on the regulation of the mTOR pathway was also tested, but no differences were observed in mTOR or in the phosphorylations of p70S6K and RPS6 (Figure 6B).

Thus, the administration of tideglusib did not activate the Wnt or mTOR pathways in fibroblasts or CD56-cells. The absence of effect after treatment with tideglusib in different cell types is of great interest, since it indicates that the treatment is not affecting other tissues avoiding undesired adverse effects.

## Claims

1. A compound of formula (I): 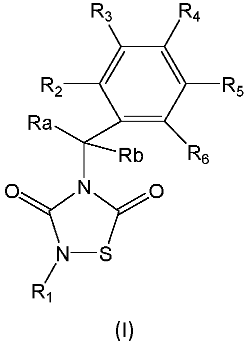 wherein:
R₁ is a substituted or unsubstituted aryl group or an alkyl group substituted with at least one aryl group;
Rₐ, R_{b}, R₂, R₃, R₄, R₅, R₆ are independently selected from hydrogen, substituted or unsubstituted alkyl, substituted or unsubstituted cycloalkyl, substituted or unsubstituted alkenyl, substituted or unsubstituted aryl, substituted or unsubstituted heterocyclyl, substituted or unsubstituted heteroaryl, -COR₇, -C(O)OR₇, -C(O)NR₇R₈ -C=NR₇, -CN, -OR₇, - OC(O)R₇, -S(O)ₜ-R₇, -NR₇R₈, -NR₇C(O)R₈, -NO₂, -N=CR₇R₈ or halogen,
t is 0, 1, 2 or 3,
R₇ and R₈ are each independently selected from hydrogen, substituted or unsubstituted alkyl, substituted or unsubstituted cycloalkyl, substituted or unsubstituted alkenyl, substituted or unsubstituted aryl, substituted or unsubstituted heterocyclyl, substituted or unsubstituted heteroaryl, substituted or unsubstituted alkoxy, substituted or unsubstituted aryloxy, halogen;
wherein Rₐ and R_{b} together can form a group =O, and wherein any pair Rₐ R₂, R₂ R₃, R₃ R₄, R₄ R₅, Rₛ R₅, R₆ R_{b}, or R₇R₈ can form together a cyclic substituent;
or a pharmaceutically acceptable salt or solvate thereof,
for its use in the treatment of limb girdle muscular dystrophy.

2. Compound for use according to claim 1, wherein R₁ is selected from:
- a C₆-C₁₀ aryl group, optionally substituted with at least one C₁-C₆ alkyl group, an aryloxy group, or an aralkyl group, or fused to a non-aromatic ring; and
- a C₁-C₃ alkyl group substituted with at least one C₆-C₁₀ aryl group, wherein the aryl group is optionally substituted with at least one C₁-C₃ alkyl group or C₁-C₃ alkoxy group.

3. Compound for use according to claims 1 or 2, wherein R₁ is a C₆-C₁₀ aryl group optionally substituted with at least one C₁-C₆ alkyl group, an aryloxy group, or an aralkyl group, or fused to a non-aromatic ring.

4. Compound for use according to claim 3, wherein R₁ is a non-substituted C₆-C₁₀ aryl group.

5. Compound for use according to any one of preceding claims, wherein R₁ is selected from:

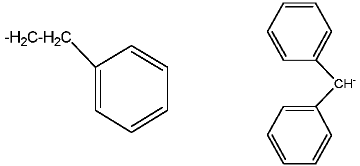

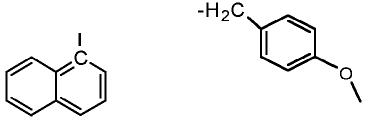

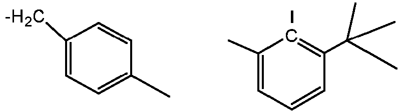

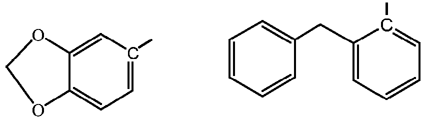

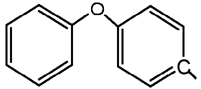

6. Compound for use according to any one of preceding claims, wherein R₁ is a naphthyl group.

7. Compound for use according to any one of the preceding claims, wherein Rₐ and R_{b} are H.

8. Compound for use according to any one of the preceding claims, wherein R₂, R₃, R₄, R₅, R₆ are independently selected from hydrogen, substituted or unsubstituted alkyl, COR₇, -C(O)OR₇, - OR₇, -NR₇R₈, or halogen, wherein R₇ and R₈ are each independently selected from hydrogen, substituted or unsubstituted alkyl, substituted or unsubstituted cycloalkyl, substituted or unsubstituted alkenyl, substituted or unsubstituted aryl, substituted or unsubstituted heterocyclyl, substituted or unsubstituted heteroaryl, substituted or unsubstituted alkoxy, substituted or unsubstituted aryloxy, halogen.

9. Compound for use according to any one of the preceding claims, wherein R₂, R₃, R₄, R₅, R₆ are H.

10. Compound for use according to any one of the preceding claims, wherein Rₐ, R_{b}, R₂, R₃, R₄, R₅ and R₆ are H.

11. Compound for use according to any one of the preceding claims, selected from: 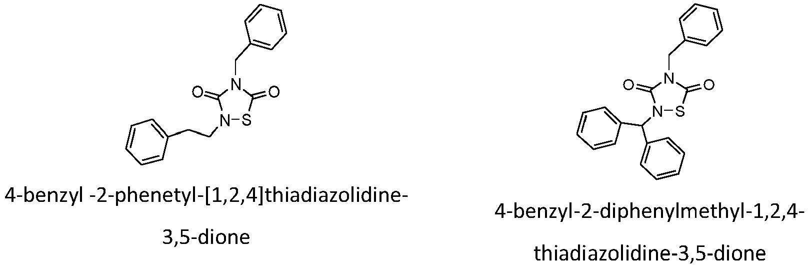 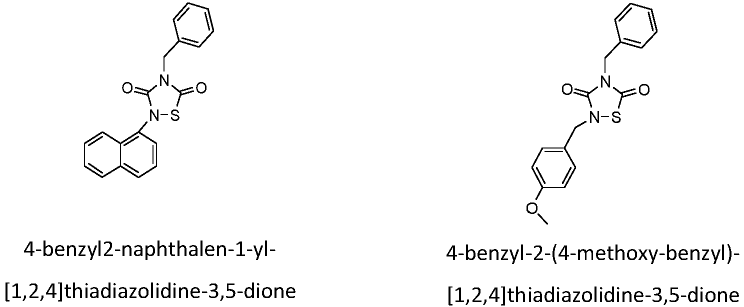 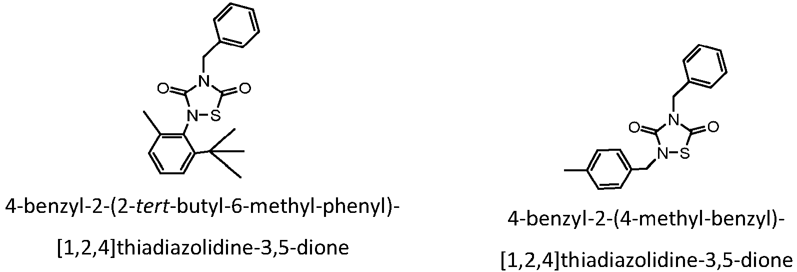 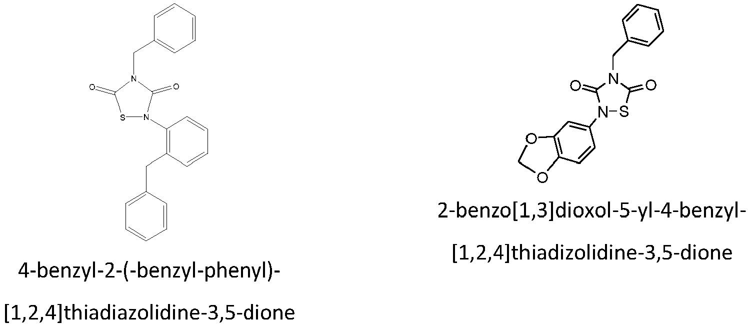 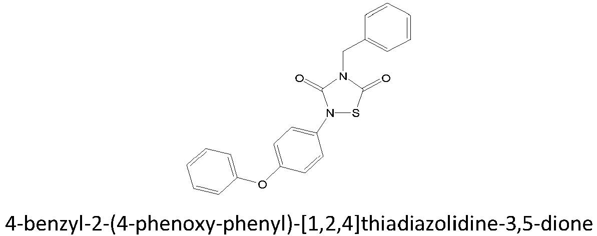 and their salts or solvates.

12. Compound for use according to any one of the preceding claims, which is: 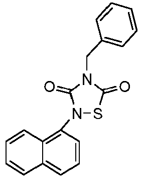 or a salt or solvate thereof.

13. Compound for use according to any one of the preceding claims, wherein the limb girdle muscular dystrophy is a recessive limb girdle muscular dystrophy selected from LGMD R1 calpain3-related (LGMDR1 or calpainopathy), LGMD R2 dysferlin-related (LGMDR2), LGMD R3 α-sarcoglycan-related (LGMDR3), LGMD R4 β-sarcoglycan-related (LGMDR4), LGMD R5 γ -sarcoglycan-related (LGMDR5), LGMD R6 δ-sarcoglycan-related (LGMDR6), LGMD R7 telethonin-related (LGMDR7), LGMD R8 TRIM 32-related (LGMDR8), LGMD R9 FKRP-related (LGMDR9), LGMD R10 titin-related (LGMDR10), LGMD R11 POMT1-related (LGMDR11), LGMD R12 anoctamin5-related (LGMDR12), LGMD R13 Fukutin-related (LGMDR13), LGMD R14 POMT2-related (LGMDR14), LGMD R15 POMGnT1-related (LGMDR15), LGMD R16 α-dystroglycan-related (LGMDR16), LGMD R17plectin-related (LGMDR17), LGMD R18TRAPPC11-related (LGMDR18), LGMD R19GMPPB-related (LGMDR19), LGMD R20ISPD-related (LGMDR20), LGMD R21POGLUT1-related (LGMDR21), LGMD R22collagen6-related (LGMDR22), LGMD R23laminin α2-related (LGMDR23), and LGMD R24POMGNT2-related (LGMDR24); or a dominant limb girdle muscular dystrophy selected from LGMD D1 DNAJB6-related (LGMD D1), LGMD D2 TNP03-related (LGMD D2), LGMD D3 HNRNPDL-related (LGMD D3), LGMD D4 calpain3-related (LGMD D4) and LGMD D5 collagen6-related (LGMD D5).

14. Compound for use according to claim 13, wherein the limb girdle muscular dystrophy is limb-girdle muscular dystrophy R1 calpain 3-related.

15. Compound for use according to any one of the preceding claims, which is used in combination with one or more active ingredients to provide a combination therapy, wherein the other active ingredients may form part of the same composition, or be provided as a separate composition for administration at the same time or at different time.

## Patentansprüche

1. Eine Verbindung der Formel (I): 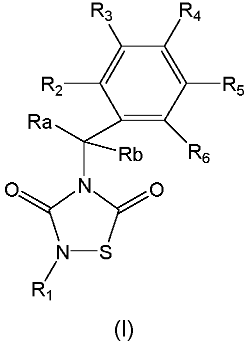 worin:
R₁ ist eine substituierte oder unsubstituierte Arylgruppe oder eine Alkylgruppe, die mit mindestens einer Arylgruppe substituiert ist;
Rₐ, R_{b}, R₂, R₃, R₄, Rs, R₆ werden unabhängig voneinander ausgewählt aus Wasserstoff, substituiertem oder unsubstituiertem Alkyl, substituiertem oder unsubstituiertem Cycloalkyl, substituiertem oder unsubstituiertem Alkenyl, substituiertem oder unsubstituiertem Aryl, substituiertem oder unsubstituiertem Heterocyclyl, substituiertem oder unsubstituiertem Heteroaryl, -COR₇, -C(O)OR₇, -C(O)NR₇R₈, -C=NR₇, -CN, -OR₇, -OC(O)R₇, -S(O)ₜ-R₇, -NR₇R₈, -NR₇C(O)R₈, -NO₂, -N=CR₇R₈ oder Halogen,
t ist 0, 1, 2 oder 3,
R₇ und R₈ sind jeweils unabhängig voneinander ausgewählt aus Wasserstoff, substituiertem oder unsubstituiertem Alkyl, substituiertem oder unsubstituiertem Cycloalkyl, substituiertem oder unsubstituiertem Alkenyl, substituiertem oder unsubstituiertem Aryl, substituiertem oder unsubstituiertem Heterocyclyl, substituiertem oder unsubstituiertem Heteroaryl, substituiertem oder unsubstituiertem Alkoxy, substituiertem oder unsubstituiertem Aryloxy, Halogen;
wobei Ra und R_{b} zusammen eine Gruppe =O bilden können, und wobei jedes Paar Rₐ R₂, R₂ R₃, R₃ R₄, R₄ R₅, R₅ R₆, R₆ R_{b} oder R₇R₈ zusammen einen zyklischen Substituenten bilden kann;
oder ein pharmazeutisch verträgliches Salz oder Solvat davon,
zur Verwendung in der Behandlung von Gliedergürtel-Muskeldystrophie.

2. Verbindung zur Verwendung nach Anspruch 1, wobei R₁ ausgewählt ist aus:
- eine C₆-C₁₀-Arylgruppe, gegebenenfalls substituiert mit mindestens einer C₁-C₆-Alkylgruppe, einer Aryloxygruppe oder einer Aralkylgruppe, oder mit einem nichtaromatischen Ring fusioniert; und
- eine C₁-C₃-Alkylgruppe, substituiert mit mindestens einer C₆-C₁₀₋Arylgruppe, wobei die Arylgruppe gegebenenfalls mit mindestens einer C₁-C₃-Alkylgruppe oder C₁-C₃-Alkoxygruppe substituiert ist.

3. Verbindung zur Verwendung nach Anspruch 1 oder 2, wobei R₁ eine C₆-C₁₀-Arylgruppe ist, die gegebenenfalls mit mindestens einer C₁-C₆-Alkylgruppe, einer Aryloxygruppe oder einer Aralkylgruppe substituiert oder mit einem nichtaromatischen Ring verschmolzen ist.

4. Verbindung zur Verwendung nach Anspruch 3, wobei R₁ eine nicht substituierte C₆-C₁₀-Arylgruppe ist.

5. Verbindung zur Verwendung nach einem der vorhergehenden Ansprüche, wobei R₁ ausgewählt ist aus:

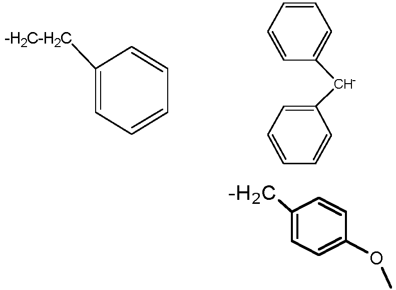

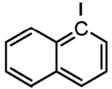

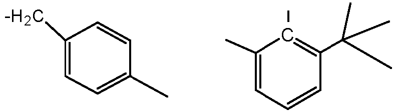

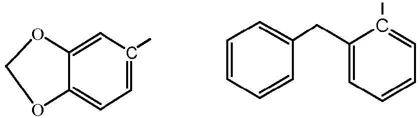

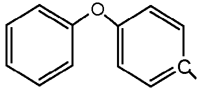

6. Verbindung zur Verwendung nach einem der vorhergehenden Ansprüche, wobei R₁ eine Naphthylgruppe ist.

7. Verbindung zur Verwendung nach einem der vorhergehenden Ansprüche, wobei Ra und R_{b} H sind.

8. Verbindung zur Verwendung nach einem der vorhergehenden Ansprüche, wobei R₂, R₃, R₄, Rs, R₆ unabhängig voneinander ausgewählt sind aus Wasserstoff, substituiertem oder unsubstituiertem Alkyl, COR₇, -C(O)OR₇, -OR₇, -NR₇R₈ oder Halogen, wobei R₇ und Rs jeweils unabhängig voneinander ausgewählt sind aus Wasserstoff, substituiertem oder unsubstituiertem Alkyl, substituiertem oder unsubstituiertem Cycloalkyl, substituiertem oder unsubstituiertem Alkenyl, substituiertes oder unsubstituiertes Aryl, substituiertes oder unsubstituiertes Heterocyclyl, substituiertes oder unsubstituiertes Heteroaryl, substituiertes oder unsubstituiertes Alkoxy, substituiertes oder unsubstituiertes Aryloxy, Halogen.

9. Verbindung zur Verwendung nach einem der vorhergehenden Ansprüche, wobei R₂, R₃, R₄, R₅, R₆ H sind.

10. Verbindung zur Verwendung nach einem der vorhergehenden Ansprüche, wobei Rₐ, R, _{b}, R₂, R₃, R₄, R₅ und R₆ H sind.

11. Verbindung zur Verwendung nach einem der vorhergehenden Ansprüche, ausgewählt aus: 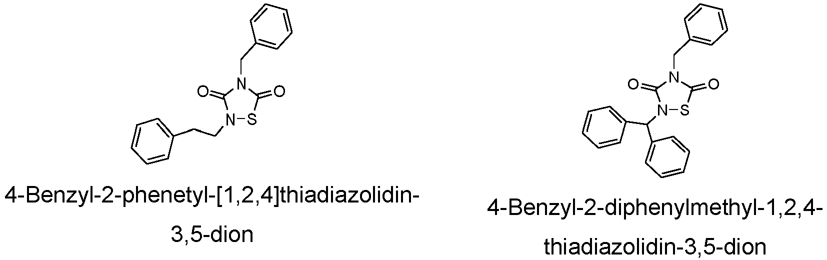 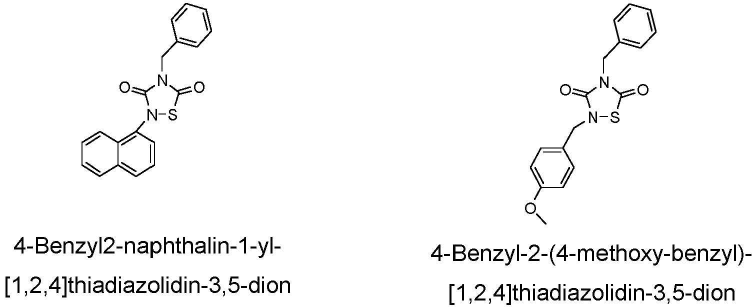 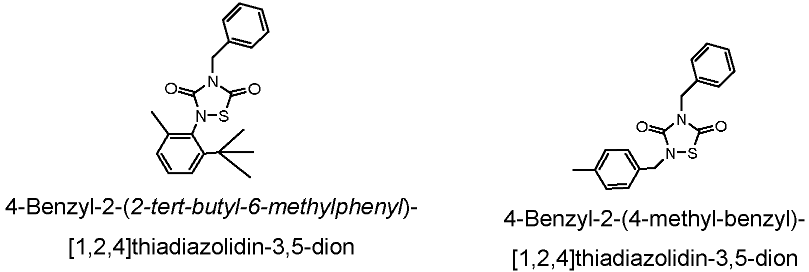 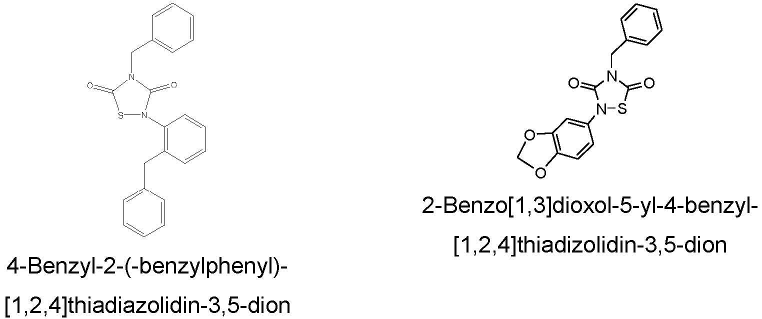 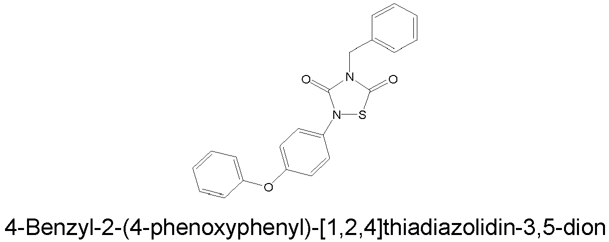 und deren Salze oder Solvate.

12. Verbindung zur Verwendung nach einem der vorhergehenden Ansprüche, d. h. 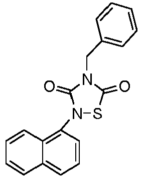 oder ein Salz oder Solvat davon.

13. Verbindung zur Verwendung nach einem der vorhergehenden Ansprüche, wobei die Gliedergürtel-Muskeldystrophie eine rezessive Gliedergürtel-Muskeldystrophie ist, ausgewählt aus LGMD R1 Calpain3-bezogen (LGMDR1 oder Calpainopathie), LGMD R2 Dysferlin-verwandt (LGMDR2), LGMD R3 α-Sarkoglykan-verwandt (LGMDR3), LGMD R4 β-Sarkoglykan-verwandt (LGMDR4), LGMD R5 γ-Sarkoglykan-verwandt (LGMDR5), LGMD R6 δ-Sarkoglykan-verwandt (LGMDR6), LGMD R7 telethonin-bezogen (LGMDR7), LGMD R8 TRIM 32-bezogen (LGMDR8), LGMD R9 FKRP-bezogen (LGMDR9), LGMD R10 Titin-bezogen (LGMDR10), LGMD R11 POMT1-bezogen (LGMDR11), LGMD R12 Anoctamin5-bezogen (LGMDR12), LGMD R13 Fukutin-bezogen (LGMDR13), LGMD R14 POMT2-bezogen (LGMDR14), LGMD R15 POMGnT1-bezogen (LGMDR15), LGMD R16 α-Dystroglykan-bezogen (LGMDR16), LGMD R17plektin-bezogen (LGMDR17), LGMD R18TRAPPC11-bezogen (LGMDR18), LGMD R19GMPPB-bezogen (LGMDR19), LGMD R20ISPD-bezogen (LGMDR20), LGMD R21POGLUT1-bezogen (LGMDR21), LGMD R22Kollagen6-bezogen (LGMDR22), LGMD R23laminin α2-bezogen (LGMDR23) und LGMD R24POMGNT2-bezogen (LGMDR24); oder eine dominante Muskeldystrophie des Gliedmaßengürtels, ausgewählt aus LGMD D1 DNAJB6-bezogen (LGMD D1), LGMD D2 TNP03-bezogen (LGMD D2), LGMD D3 HNRNPDL-bezogen (LGMD D3), LGMD D4 Calpain3-bezogen (LGMD D4) und LGMD D5 Kollagen6-bezogen (LGMD D5).

14. Verbindung zur Verwendung nach Anspruch 13, wobei die Gliedergürtel-Muskeldystrophie mit der Gliedergürtel-Muskeldystrophie R₁ calpain 3-bezogen ist.

15. Verbindung zur Verwendung nach einem der vorhergehenden Ansprüche, die in Kombination mit einem oder mehreren Wirkstoffen verwendet wird, um eine Kombinationstherapie bereitzustellen, wobei die anderen Wirkstoffe Teil derselben Zusammensetzung sein können oder als separate Zusammensetzung zur Verabreichung zur gleichen Zeit oder zu unterschiedlichen Zeitpunkten bereitgestellt werden können.

## Revendications

1. Composé de formule (I) : 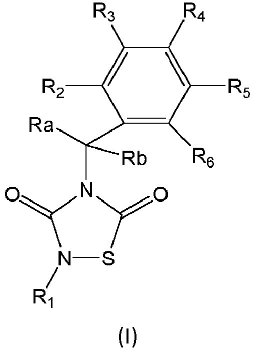 dans lequel :
R₁ est un groupe aryle substitué ou non substitué ou un groupe alkyle substitué par au moins un groupe aryle,
Rₐ, R_{b}, R₂, R₃, R₄, R₅, R₆ sont choisis indépendamment parmi l'hydrogène, un alkyle substitué ou non substitué, un cycloalkyle substitué ou non substitué, un alcényle substitué ou non substitué, un aryle substitué ou non substitué, un hétérocyclyle substitué ou non substitué, un hétéroaryle substitué ou non substitué, -COR₇, -C(O)OR₇, - C(O)NR₇R₈ -C=NR₇, -CN, -OR₇, -OC(O)R₇, -S(O)ₜ-R₇, -NR₇R₈, -NR₇C(O)R₈, -NO₂, - N=CR₇R₈ ou un halogène,
t est 0, 1, 2 ou 3,
R₇ et R₈ sont chacun choisis indépendamment parmi l'hydrogène, un alkyle substitué ou non substitué, un cycloalkyle substitué ou non substitué, un alcényle substitué ou non substitué, un aryle substitué ou non substitué, un hétérocyclyle substitué ou non substitué, un hétéroaryle substitué ou non substitué, un alcoxy substitué ou non substitué, un aryloxy substitué ou non substitué, un halogène,
dans lequel Rₐ et R_{b} peuvent former ensemble un groupe =O, et dans lequel toute paire Rₐ R₂, R₂ R₃, R₃ R₄,, R₄ R₅, R₅ R₆, R₆ R_{b} ou R₇ R₈ peut former ensemble un substituant cyclique,
ou un sel ou solvate de celui-ci pharmaceutiquement acceptable,
pour son utilisation dans le traitement de la dystrophie musculaire des ceintures.

2. Composé pour une utilisation selon la revendication 1, dans lequel R₁ est choisi parmi :
- un groupe aryle en C₆ à C₁₀, optionnellement substitué par au moins un groupe alkyle en C₁ à C₆, un groupe aryloxy ou un groupe aralkyle, ou fusionné à un cycle non aromatique, et
- un groupe alkyle en C₁ à C₃ substitué par au moins un groupe aryle en C₆ à C₁₀, dans lequel le groupe aryle est optionnellement substitué par au moins un groupe alkyle en C₁ à C₃ ou un groupe alcoxy en C₁ à C₃.

3. Composé pour une utilisation selon la revendication 1 ou 2, dans lequel R₁ est un groupe aryle en C₆ à C₁₀ optionnellement substitué par au moins un groupe alkyle en C₁ à C₆, un groupe aryloxy ou un groupe aralkyle, ou fusionné à un cycle non aromatique.

4. Composé pour une utilisation selon la revendication 3, dans lequel R₁ est un groupe aryle en C₆ à C₁₀ non substitué.

5. Composé pour une utilisation selon l'une quelconque des revendications précédentes, dans lequel R₁ est choisi parmi :

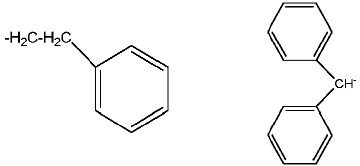

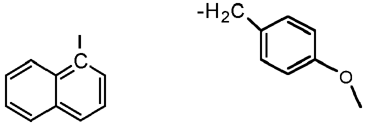

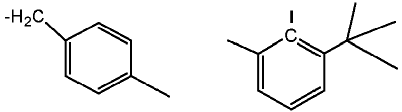

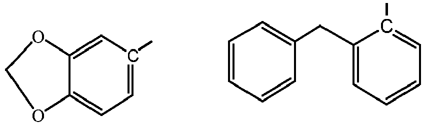

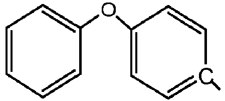

6. Composé pour une utilisation selon l'une quelconque des revendications précédentes, dans lequel R₁ est un groupe naphtyle.

7. Composé pour une utilisation selon l'une quelconque des revendications précédentes, dans lequel Rₐ et R_{b} sont H.

8. Composé pour une utilisation selon l'une quelconque des revendications précédentes, dans lequel R₂, R₃, R₄, R₅, R₆ sont choisis indépendamment parmi l'hydrogène, un alkyle substitué ou non substitué, COR₇, -C(O)OR₇, -OR₇, -NR₇R₈, ou un halogène, dans lequel R₇ et R₈sont chacun choisis indépendamment parmi l'hydrogène, un alkyle substitué ou non substitué, un cycloalkyle substitué ou non substitué, un alcényle substitué ou non substitué, un aryle substitué ou non substitué, un hétérocyclyle substitué ou non substitué, un hétéroaryle substitué ou non substitué, un alcoxy substitué ou non substitué, un aryloxy substitué ou non substitué, un halogène.

9. Composé pour une utilisation selon l'une quelconque des revendications précédentes, dans lequel R₂, R₃, R₄, R₅ et R₆ sont H.

10. Composé pour une utilisation selon l'une quelconque des revendications précédentes, dans lequel Rₐ, R_{b}, R₂, R₃, R₄, R₅ et R₆ sont H.

11. Composé pour une utilisation selon l'une quelconque des revendications précédentes, choisi parmi : 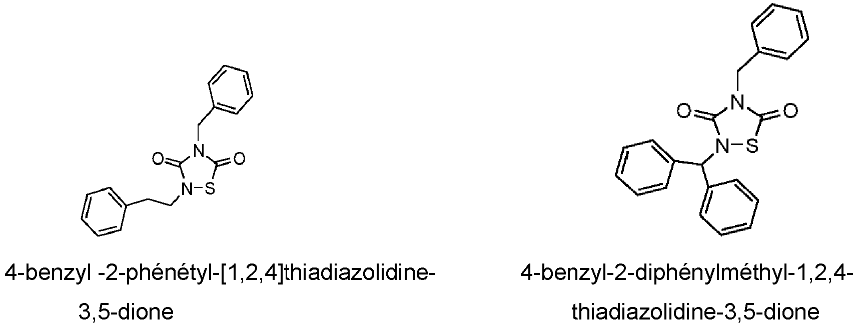 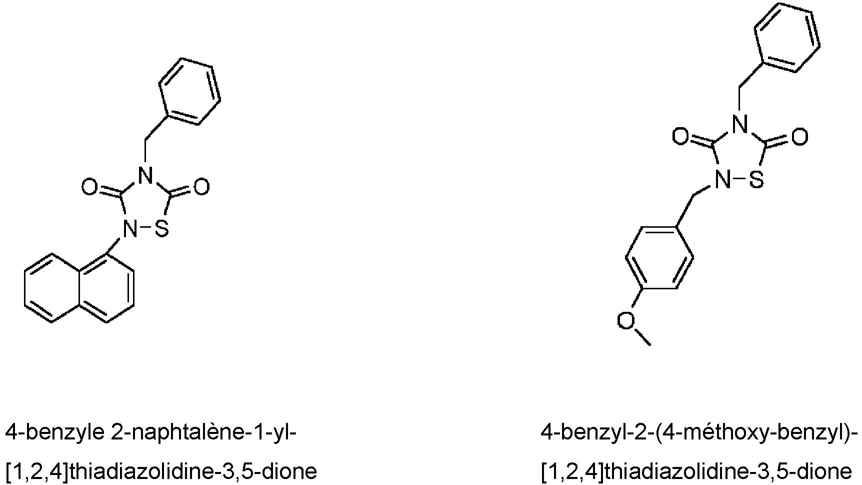 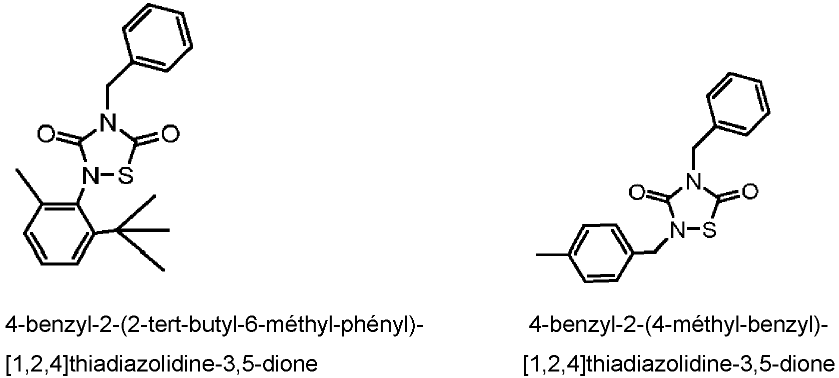 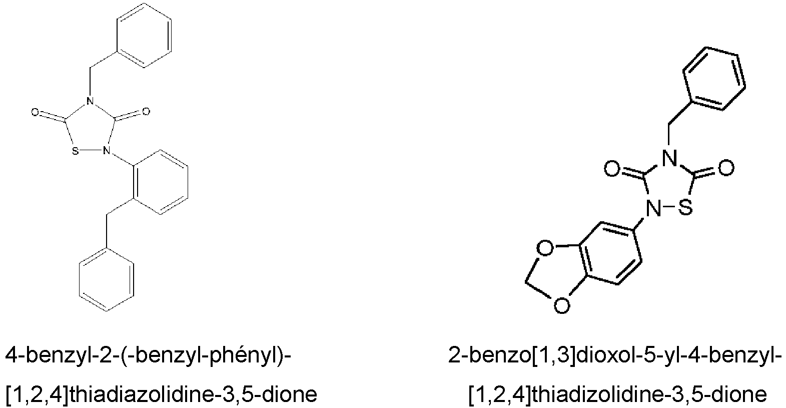 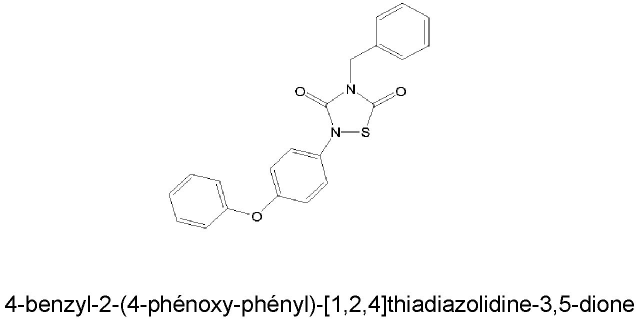 et leurs sels ou solvates.

12. Composé pour une utilisation selon l'une quelconque des revendications précédentes, qui est : 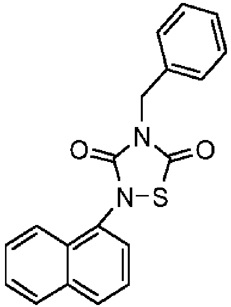 ou un sel ou solvate de celui-ci.

13. Composé pour une utilisation selon l'une quelconque des revendications précédentes, dans lequel la dystrophie musculaire des ceintures est une dystrophie musculaire des ceintures récessive choisie parmi LGMD R1 liée à la calpaïne 3 (LGMDR1 ou calpaïnopathie), LGMD R2 liée à la dysferline (LGMDR2), LGMD R3 liée au α-sarcoglycane (LGMDR3), LGMD R4 liée au β-sarcoglycane (LGMDR4), LGMD R5 liée au γ-sarcoglycane (LGMDR5), LGMD R6 liée au δ-sarcoglycane (LGMDR6), LGMD R7 liée à la téléthonine (LGMDR7), LGMD R8 liée à la TRIM 32 (LGMDR8), LGMD R9 liée au FKRP (LGMDR9), LGMD R10 liée à la titine (LGMDR10), LGMD R11 liée à la POMT1 (LGMDR11), LGMD R12 liée à l'anoctamine 5 (LGMDR12), LGMD R13 liée à la fukutine (LGMDR13), LGMD R14 liée à la POMT2 (LGMDR14), LGMD R15 liée au POMGnT1 (LGMDR15), LGMD R16 liée au α-dystroglycane (LGMDR16), LGMD R17 liée à la plectine (LGMDR17), LGMD R18 liée au TRAPPC11 (LGMDR18), LGMD R19 liée au GMPPB (LGMDR19), LGMD R20 liée à l'ISPD (LGMDR20), LGMD R21 liée au POGLUT1 (LGMDR21), LGMD R22 liée au collagène 6 (LGMDR22), LGMD R23 liée à la laminine a2 (LGMDR23) et LGMD R24 liée au POMGNT2 (LGMDR24), ou une dystrophie musculaire des ceintures dominante choisie parmi LGMD D1 liée à la DNAJB6 (LGMD D1), LGMD D2 liée à la TNP03 (LGMD D2), LGMD D3 liée au HNRNPDL (LGMD D3), LGMD D4 liée à la calpaïne 3 (LGMD D4) et LGMD D5 liée au collagène 6 (LGMD D5).

14. Composé pour une utilisation selon la revendication 13, dans lequel la dystrophie musculaire des ceintures est une dystrophie musculaire des ceintures R1 liée à la calpaïne 3.

15. Composé pour une utilisation selon l'une quelconque des revendications précédentes, qui est utilisé en combinaison avec un ou plusieurs ingrédients actifs pour fournir une thérapie de combinaison, dans laquelle les autres ingrédients actifs peuvent faire partie de la même composition, ou être fournis sous forme d'une composition séparée en vue d'une administration simultanée ou à différents moments.
